# DEMANDE DE BREVET EUROPEEN

(11) **EP 3 673 764 A1**
(43) Date de publication de la demande: **01.07.2020**
(21) Numéro de dépôt: 19218522.1
(22) Date de dépôt: 20.12.2019
(51) Int. Cl.: A45D 26/00, B01F 7/16, B01F 15/06, B01F 7/00, B01F 13/00

(54) **PROCÉDÉ ET DISPOSITIF DE FABRICATION D'UNE COMPOSITION ÉPILATOIRE**

(30) Priorité: 27.12.2018 FR 1874265
(71) Demandeur: SEB S.A., 69130 Ecully (FR)
(72) Inventeur: DELHOM, Jérémy, 69004 LYON (FR); HOFLEITNER, Céline, 69003 LYON (FR)
(74) Mandataire: Bourrières, Patrice

(57) **Abrégé**

La présente invention concerne un appareil pour fabrication de cire à épiler (1), comprenant :
- une cuve (2) destinée à recevoir une préparation pour cire à épiler ;
- un couvercle (3) prévu pour fermer la cuve (2) ;
- un bloc moteur (4) et un arbre d'entrainement (5) configuré pour être entrainé en rotation par ledit bloc moteur (4) ;
- au moins une pâle (61) prévue pour être mise en rotation par l'arbre d'entrainement (5), l'au moins une pâle (61) étant agencée dans la cuve (2) pour permettre un mélange de la préparation pour cire à épiler ;
- un système de chauffage (7) configuré pour un chauffage de la cuve (2) ; et
- un système de refroidissement (8) configuré pour un refroidissement de la cuve (2).

L'invention concerne également un procédé de fabrication de cire à épiler, possible notamment avec un tel appareil.

## Description

### DOMAINE DE L'INVENTION

La présente invention concerne le domaine des appareils et des procédés de préparation de cire à épiler, et notamment de cire à base de sucre.

### ETAT DE LA TECHNIQUE

Le procédé de mise en œuvre de cire à base de sucre est bien connu et utilisé dans des instituts de beauté ou par des utilisateurs particuliers.

Le document FR2777458 décrit par exemple une composition épilatoire à base de miel et de jus de citron, conçue de manière à être aisément appliquée à la main, et un procédé de fabrication de cette composition, comprenant les étapes de chauffage et de mélange jusqu'à une température déterminée, puis un séchage de la composition. Cependant, ce procédé n'est pas simple à mettre en œuvre par des utilisateurs particuliers, puisqu'il existe de nombreux paramètres dans le procédé de fabrication et dans les compositions à utiliser, qui sont peu maitrisés et peu maitrisables par l'utilisateur. En outre, le miel peut avoir un coût important pour un utilisateur.

Le document FR2541894 décrit un procédé de préparation d'une composition comprenant du sucre, de l'eau et du jus de citron, de faible coût et peu irritante pour la peau d'un utilisateur. Cependant, ce procédé reste encore une fois difficile à réaliser par un utilisateur, sans connaissance précise des paramètres définissant le procédé de fabrication.

Le procédé de fabrication d'une cire à épiler à base de sucre, d'eau et de jus de citron est très difficile à maitriser car il dépend de nombreux facteurs, y compris des facteurs liés à l'environnement dans lequel la composition épilatoire est préparée.

De manière générale, lors d'une utilisation par un utilisateur à domicile, la cire au sucre est achetée en pot directement en grande surface par exemple, et est réchauffée avant chaque utilisation, par exemple au bain-marie ou au micro-onde. A titre illustratif, le document EP1568294 décrit un dispositif de chauffage de cire à épiler à l'usage d'utilisateurs particuliers permettant le contrôle de la température de chauffage selon différents modes. Cependant, ce dispositif permet de chauffer une cire déjà préparée et contenue dans un pot, qui sera placé à l'intérieur du dispositif.

### EXPOSE DE L'INVENTION

Un but de l'invention est de remédier au moins en partie aux inconvénients précités, et notamment de proposer un procédé de fabrication de cire à épiler simple, destiné à être mis en œuvre par un appareil de façon automatisée, en prenant en compte différents facteurs ambiants.

A cette fin, on propose un appareil de fabrication de cire à épiler, comprenant :
- une cuve destinée à recevoir une préparation pour cire à épiler ;
- un couvercle prévu pour fermer la cuve ;
- un bloc moteur et un arbre d'entrainement configuré pour être entrainé en rotation par ledit bloc moteur ;
- au moins une pâle prévue pour être mise en rotation par l'arbre d'entrainement, l'au moins une pâle étant agencée dans la cuve pour permettre un mélange de la préparation pour cire à épiler ;
- un système de chauffage configuré pour un chauffage de la cuve ; et
- un système de refroidissement configuré pour un refroidissement de la cuve.

L'appareil de fabrication de cire à épiler peut comprendre les caractéristiques suivantes, prises seules ou en combinaison :
- l'appareil de fabrication de cire à épiler est portatif ;
- la cuve comprend un fond et des parois latérales, le système de chauffage étant agencé pour un chauffage du fond de la cuve et le système de refroidissement étant agencé pour un refroidissement des parois latérales de la cuve ;
- la cuve et/ou l'au moins une pâle sont amovibles ;
- l'appareil de fabrication de cire à épiler comprend en outre une base ayant une portion inférieure et une portion supérieure couplée à la portion inférieure, la portion supérieure étant destinée à recevoir la cuve de sorte qu'un espace de refroidissement est défini entre une surface latérale extérieure de la cuve et une surface intérieure de la portion supérieure ;
- le bloc moteur, le système de chauffage et le système de refroidissement sont agencés dans la portion inférieure ;
- la portion inférieure est formée par une première coque et la portion supérieure est formée par une deuxième coque venant prolonger la première coque ;
- la portion inférieure et/ou la portion supérieure comprend des ouvertures configurées pour permettre une circulation d'air dans l'espace de refroidissement ;
- le couvercle comprend une pluralité d'ouvertures pour une circulation d'air entre l'intérieur de la cuve et l'extérieur, et un système de blocage configuré pour verrouiller une fermeture de la cuve ;
- l'appareil de fabrication de cire à épiler comprend en outre un élément de protection thermique agencé pour isoler thermiquement le système de chauffage du bloc moteur et/ou du système de refroidissement ;
- le système de refroidissement, le bloc moteur et l'arbre sont alignés selon un axe d'alignement correspondant à l'axe de rotation de l'arbre d'entrainement ;
- le bloc moteur est en outre configuré pour commander le système de refroidissement ;
- le système de refroidissement comprend un ventilateur, ledit ventilateur étant agencé pour diriger de l'air en direction de la cuve ;
- la cuve a une surface intérieure dotée d'un revêtement antiadhésif, de préférence un revêtement à base de PTFE ;
- l'appareil de fabrication de cire à épiler comprend en outre un système de pesée, configuré pour peser un contenu de la cuve ;
- l'appareil de fabrication de cire à épiler comprend en outre un capteur de température interne permettant de déterminer la température à l'intérieur de la cuve, et un contrôleur configuré pour contrôler le système de chauffage, le système de refroidissement et/ou le bloc moteur en fonction de la température à l'intérieur de la cuve ;
- l'appareil de fabrication de cire à épiler comprend en outre un capteur de température externe permettant de déterminer la température à l'extérieur de l'appareil, et un système d'avertissement prévu pour donner des informations à un utilisateur de l'appareil en fonction de la température à l'extérieur de l'appareil.

On propose également un procédé de fabrication de cire à épiler comprenant les étapes successives suivantes :
a) incorporation d'une préparation pour cire à épiler dans une cuve, la préparation comprenant un mélange d'une matière majoritairement composée de glucides simples, d'une solution acidifiante et d'eau (par exemple un mélange de sucre, d'eau et de jus de citron) ;
b) chauffage et mélange de ladite préparation jusqu'à une première température, la première température étant comprise entre 125°C et 140°C, et de préférence entre 130°C et 135°C,
c) refroidissement forcé et mélange de la préparation jusqu'à une deuxième température, la deuxième température étant de l'ordre de 70°C,
d) refroidissement forcé sans mélange de la préparation jusqu'à une troisième température, la troisième température étant de l'ordre de 45°C.

Le procédé de fabrication de cire à épiler peut comprendre les caractéristiques suivantes, prises seules ou en combinaison :
- l'étape d) est suivie d'une étape e) de maintien de la préparation à la troisième température ;
- l'étape b) est effectuée pendant une durée d'environ 15min ;
- les étapes c) et d) ont une durée totale inférieure à 20min ;
- le procédé de fabrication de cire à épiler comprend en outre un contrôle continu de la température à l'intérieur de la cuve, la succession des étapes b) à d) étant automatiquement effectuée en fonction de ladite température à l'intérieur de la cuve ;

Par exemple, la préparation utilisée pour l'étape a) est un mélange comprenant 55% de sucre, 10% d'eau et 35% de jus de citron pour une température ambiante comprise entre 20°C et 23°C, ou un mélange comprenant 55% de sucre, 13% d'eau et 32% de jus de citron pour une température ambiante comprise entre 24°C et 27°C, ou un mélange comprenant 55% de sucre, 17% d'eau et 28% de jus de citron pour une température ambiante comprise entre 28°C et 30°C.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
La Figure 1 illustre une vue d'ensemble d'un appareil pour fabrication de cire à épiler selon un exemple de réalisation de l'invention.
La Figure 2 illustre une vue en coupe de l'appareil pour fabrication de cire à épiler selon l'exemple de réalisation de la figure 1.
La Figure 3 illustre une vue en perspective éclatée de l'appareil pour fabrication de cire à épiler selon l'exemple de réalisation de la figure 1.
La Figure 4 est un organigramme d'étapes d'un procédé de fabrication de cire à épiler pouvant être mis en œuvre par un appareil tel que représenté sur la figure 1.

Seuls les éléments nécessaires à la compréhension de l'invention ont été représentés. Pour faciliter la lecture des dessins, les éléments similaires portent des références identiques sur l'ensemble des figures.

### DESCRIPTION DETAILLEE DE L'INVENTION

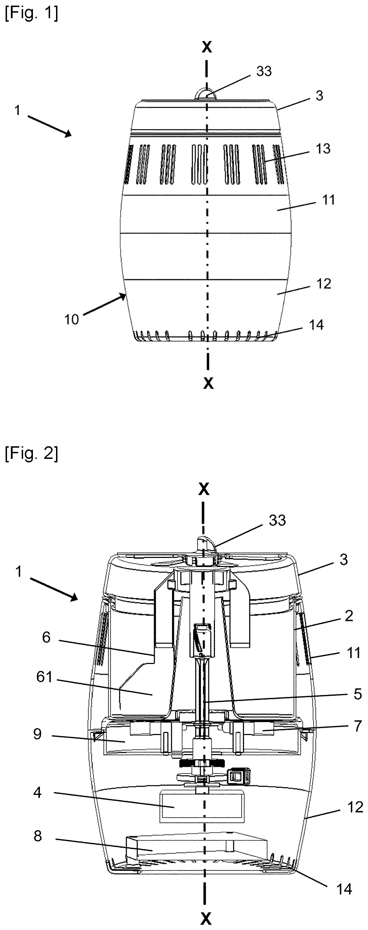

La figure 1 illustre une vue d'ensemble un appareil pour fabrication de cire à épiler 1 selon un exemple de réalisation.

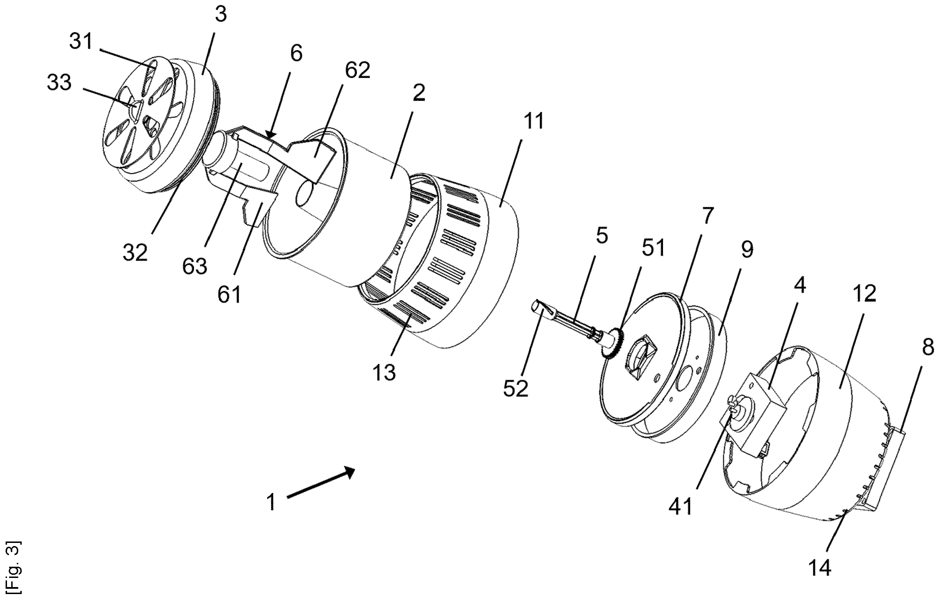

Comme cela est illustré sur la vue en coupe de la figure 2 et la vue en perspective éclatée de la figure 3, l'appareil pour fabrication de cire à épiler 1 (nommé par la suite appareil 1) comprend une cuve 2, destinée à recevoir des composants permettant de réaliser une préparation pour cire à épiler. Typiquement, comme il sera détaillé par la suite, les composants peuvent comprendre du sucre, de l'eau et du jus de citron, de sorte à réaliser une cire à épiler naturelle et à faible coût. La cuve 2 est de préférence composée d'un matériau permettant une bonne conduction thermique, par exemple un métal ou un alliage métallique. De manière avantageuse, une surface interne de la cuve 2 comprend un matériau antiadhésif, ou est revêtue par un matériau antiadhésif, par exemple du PTFE (téflon), de sorte à faciliter un nettoyage de la cuve 2 après utilisation.

Par exemple, la cuve 2 a une forme sensiblement cylindrique de révolution autour d'un axe de révolution, appelé également axe longitudinal X-X. La cuve 2 comprend un fond et des parois latérales.

De préférence, la cuve 2 a un diamètre inférieur à 20 cm, typiquement un diamètre d'environ 15 cm, et a une hauteur inférieure à 12 cm, typiquement environ 10 cm. Les dimensions globales de l'appareil 1 sont inférieures à une hauteur de 25 cm et à un diamètre de 15 cm (par exemple une hauteur de 24.4 cm et un diamètre de 14.5 cm), de sorte que l'appareil 1 soit compact, et ainsi qu'il n'occupe pas un volume trop grand lors d'un stockage.

De préférence, l'appareil pour fabrication de cire à épiler 1 est adapté à une utilisation individuelle, ses dimensions, notamment celles de la cuve, étant adaptées en conséquence.

De préférence, l'appareil pour fabrication de cire à épiler 1 est un appareil portatif. Ses dimensions sont donc également prévues pour garantir cette portabilité, afin qu'il puisse par exemple être mis dans un sac de voyage. La masse de l'appareil 1 est par exemple inférieure à 3 kg, de préférence inférieure à 2.5 kg, de sorte à garantir une portabilité de l'appareil et de limiter des risques pour un utilisateur. L'appareil pour fabrication de cire à épiler 1 comprend en outre un système de mélange 6, destiné à mélanger et malaxer la préparation pour cire à épiler qui peut être fabriquée par le présent appareil.

Le système de mélange 6 comprend au moins une pâle 61, disposée à l'intérieur de la cuve 2 et configurée pour mélanger la préparation pour cire à épiler. Par exemple, le système de mélange 6 comprend deux pâles (61,62), disposées symétriquement autour d'une portion centrale configurée pour être entrainée en rotation par un arbre d'entrainement 5.

De préférence, le système de mélange 6 comprenant l'au moins une pâle 61 et la cuve 2 sont amovibles, de sorte à faciliter un nettoyage de l'appareil 1 après utilisation.

La pâle (61,62) peut comprendre une surface plane inclinée par rapport au fond de la cuve 2. La surface plane inclinée est dimensionnée de sorte à malaxer ou mélanger la préparation pour cire à épiler contenue dans la cuve 2, typiquement en raclant des parois internes de la cuve 2.

De préférence, la pâle (61,62) peut être formée dans un matériau polymère, par exemple à base de silicone, de sorte à pouvoir épouser les parois internes de la cuve 2 lors d'un fonctionnement de l'appareil 1 pendant une phase de mélange. Un couvercle 3 amovible est disposé sur la cuve 2, de sorte à recouvrir la cuve 2, afin de limiter des échanges thermiques avec l'extérieur et d'éviter des éclaboussures au cours d'une utilisation de l'appareil 1. De préférence, le couvercle 3 comprend un moyen de préhension 33, permettant de faciliter une manipulation du couvercle 3 au cours d'une utilisation.

De manière avantageuse, le couvercle 3 peut comprendre un système de blocage 32 configuré pour verrouiller une fermeture de la cuve 2, par exemple par une rotation du couvercle 3 par rapport à la cuve 2.

Typiquement, le système de blocage 32 peut comprendre une nervure selon la circonférence d'une paroi extérieure d'une portion circulaire du couvercle 3, configurée pour s'introduire dans une rainure d'une paroi intérieure d'une portion circulaire de la cuve 2.

De manière alternative, le système de blocage 32 peut comprendre un ergot disposé sur la paroi extérieure d'une portion circulaire du couvercle 3, et destiné à s'introduire dans un logement correspondant compris dans la paroi intérieure d'une portion circulaire de la cuve 2.

De préférence encore, un joint d'étanchéité, par exemple un joint torique, est agencé au niveau du système de blocage 32 pour assurer une étanchéité entre le couvercle 3 et la cuve 2.

De plus, le couvercle 3 peut comprendre une pluralité d'ouvertures 31 permettant une circulation d'air entre l'intérieur de la cuve 2 et l'extérieur.

L'appareil 1 comprend en outre un bloc moteur 4, comprenant un moteur électrique. Il est configuré pour entrainer en rotation l'arbre d'entrainement 5. L'appareil comprend également un système de chauffage 7 configuré pour un chauffage de la cuve 2, et un système de refroidissement 8 configuré pour un refroidissement de la cuve 2.

Le système de chauffage 7 peut typiquement être disposé en dessous de la cuve 2 de sorte à chauffer le fond de la cuve 2 et par conséquent la préparation à cire à épiler pouvant être contenue dans la cuve. Il s'agit par exemple d'une résistance chauffante ou d'une plaque à induction.

Dans l'exemple de réalisation représenté figure 2, le système de chauffage 7 forme un support chauffant agencé sous la cuve 2, pour un chauffage du fond de la cuve 2, tandis que le système de refroidissement 8 est agencé de sorte à permettre un refroidissement des parois latérales de la cuve 2.

De préférence, le système de refroidissement 8 est également agencé pour permettre un refroidissement des éléments chauffants du système de chauffage 7.

De préférence, l'appareil comprend en outre une base 10 ayant une portion supérieure 11 et une portion inférieure 12 couplée à la portion supérieure 11. Par exemple, la base 10 a une forme sensiblement cylindrique autour de l'axe longitudinal X-X, formant l'axe de révolution, et comprend une paroi latérale prolongeant une surface d'appui plane, comprise dans la portion inférieure 12. La portion supérieure 11 est configurée pour recevoir la cuve 2.

Par exemple, le bloc moteur 4, le système de chauffage 7 et le système de refroidissement 8 sont agencés dans la portion inférieure 12, en dessous de la portion supérieure 11.

Un espace de refroidissement est par exemple défini entre une surface latérale extérieure de la cuve 2 et une surface intérieure de la portion supérieure 11.

De manière avantageuse, la portion inférieure 12 est formée par une première coque et la portion supérieure 11 est formée par une deuxième coque venant prolonger la première coque. La deuxième coque peut être amovible ou solidaire de la première coque. La première et la deuxième coque sont typiquement formées par un matériau plastique, par exemple par moulage, puis assemblées, par exemple emboîtées.

Un système de fixation, comprenant par exemple une ou plusieurs ventouses, peut être fixé sur une paroi externe de la portion inférieure 12 formant le fond de l'appareil 1, de sorte que lorsque l'appareil 1 est disposé sur une surface plane lors d'une utilisation, l'appareil 1 adhère à la surface plane, de sorte à limiter des risques de renversement.

De préférence, au moins la portion inférieure 12 et/ou de la portion supérieure 11 comprend des ouvertures (13,14) configurées pour permettre une circulation d'air dans l'espace de refroidissement. La circulation d'air est prévue pour engendrer un échange thermique avec la cuve 2, notamment avec les parois latérales de la cuve 2, par un phénomène de convection naturelle par exemple. La circulation d'air peut également être causée par le système de refroidissement 8, l'échange thermique avec la cuve 2 ayant alors lieu par un phénomène de convection forcée.

Si la portion inférieure 12 comprend des ouvertures 14 au niveau de la paroi formant le fond de l'appareil 1, il est préférable que ledit fond comprenne des pieds permettant de maintenir le fond à distance du support sur lequel l'appareil 1 est posé. Ces pieds peuvent par exemple être formés par le système de fixation mentionné plus haut.

Le système de refroidissement 8 peut être activé et commandé par le bloc moteur 4. De préférence, le système de refroidissement 8 est activé par un moteur propre dédié au système de refroidissement 8.

De manière avantageuse, le système de refroidissement 8 peut également causer une circulation artificielle d'air, par l'intermédiaire d'une pompe ou d'une hélice par exemple. Dans ce mode de réalisation, un échange thermique entre l'air dans l'espace de refroidissement et la cuve 2 a alors également lieu par un phénomène de convection forcée.

Dans l'exemple de réalisation représenté sur la figure 1, la portion supérieure 11 comprend une pluralité d'ouvertures 13 s'étendant selon la direction longitudinale et réparties selon la circonférence de la portion supérieure 11, et la portion inférieure 12 comprend également une pluralité d'ouvertures 14 réparties selon la circonférence de la portion inférieure 12 et s'étendant entre la paroi latérale et la surface d'appui de la base 10. Les ouvertures 14 permettent par exemple de refroidir par convection le moteur électrique du bloc moteur 4 et/ou le moteur propre dédié au système de refroidissement 8. L'air peut circuler dans l'espace compris entre les ouvertures 13 de la portion supérieure 11 et les ouvertures 14 de la portion inférieure 12, notamment dans l'espace de refroidissement défini plus haut.

Typiquement, le système de refroidissement 8 peut comprendre un ventilateur, agencé pour diriger de l'air en direction de la cuve 2. Dans un exemple de réalisation, le ventilateur peut comprendre un arbre d'entrainement secondaire commandé en rotation par le bloc moteur 4, et entrainant en rotation des hélices de ventilateur. De préférence, l'arbre d'entrainement secondaire est indépendant de l'arbre d'entrainement 5 du système de mélange 6, de sorte à découpler les fonctions de refroidissement et de mélange de l'appareil 1. Par exemple, l'arbre d'entrainement secondaire du ventilateur est mis en rotation par le moteur propre dédié.

Dans un exemple de réalisation, afin d'éviter un transfert thermique indésirable entre le système de chauffage 7 et d'autres éléments que la cuve 2, l'appareil 1 peut comprendre en outre un élément de protection thermique 9 agencé pour isoler thermiquement le système de chauffage 7 du bloc moteur 4 et/ou du système de refroidissement 8.

Comme cela est illustré sur les figures 2 et 3, un tel élément de protection thermique 9 peut typiquement être composé d'une enceinte creuse disposée entre l'élément chauffant du système de chauffage 7 et le bloc moteur 4, permettant ainsi à la fois que le système de chauffage 7 n'endommage pas le bloc moteur 4 par une surchauffe, et n'interfère pas avec le système de refroidissement 8. De préférence, l'élément de protection thermique 9 est fabriqué dans ou est recouvert par un matériau peu conducteur.

De préférence, la cuve 2 a une forme sensiblement cylindrique de révolution autour de l'axe longitudinal X-X correspondant également à l'axe de rotation de l'arbre d'entrainement 5. Par exemple, afin de faciliter un assemblage de la cuve 2 et de permettre son amovibilité, la cuve comprend en une partie centrale un cylindre interne creux, configuré pour recevoir l'arbre d'entrainement 5 et une portion cylindrique creuse 63 du système de mélange 6.

Typiquement, une première extrémité de l'arbre d'entrainement 5, située en dessous de la cuve 2, est configurée pour être entrainée en rotation par le bloc moteur 4. Par exemple, une roue dentée 51 est solidaire de la première extrémité de l'arbre d'entrainement 5, et le bloc moteur 4 comprend au moins un engrenage 41, configuré pour entrainer une rotation de la roue dentée 51, et entrainant une rotation de l'arbre d'entrainement 5.

Typiquement, une deuxième extrémité de l'arbre d'entrainement 5 est configurée pour recevoir une portion supérieure du système de mélange 6, et entrainer mécaniquement une rotation de l'au moins une pâle 61 autour de l'axe longitudinal X-X. Par exemple, la deuxième extrémité de l'arbre d'entrainement 5 présente une forme sensiblement cylindrique avec un moyen de verrouillage 52, configuré pour être inséré dans la portion sensiblement cylindrique creuse 63 du système de mélange 6, et ensuite pour bloquer une rotation entre le système de mélange 6 et l'arbre d'entrainement 5, afin que l'arbre d'entrainement 5 entraine en rotation les pâles (61,62).

Dans l'exemple de réalisation représenté sur la figure 3, la paroi interne de la portion cylindrique creuse 63 du système de mélange 6 comprend une pluralité d'ergots et deux nervures formant un pas de vis, configurées pour s'insérer dans deux rainures correspondantes présentes sur le moyen de verrouillage 52.

De préférence, le bloc moteur 4, l'arbre d'entrainement 5 et la cuve 2 sont sensiblement alignés selon l'axe longitudinal X-X, formant un axe d'alignement. Cela permet un gain de compacité de l'appareil 1.

Selon un exemple de réalisation, le système de refroidissement 8 est sensiblement aligné sur l'axe longitudinal X-X, par exemple sous le bloc moteur 4. Selon un autre exemple de réalisation, le système de refroidissement 8 est situé à proximité de la surface latérale de la cuve 2.

L'appareil 1 peut comprendre un capteur de température interne permettant de déterminer la température à l'intérieur de la cuve 2. Le capteur de température interne peut être directement disposé à l'intérieur de la cuve 2. De manière alternative, le capteur de température éventuel peut être disposé à proximité du système de chauffage 7, et déduire la température à l'intérieur de la cuve 2 à partir d'une température mesurée à proximité du système de chauffage 7. Le capteur de température interne est typiquement une thermistance à coefficient de température négatif (CTN).

De préférence, l'appareil 1 comprend un thermostat électronique configuré pour maintenir la cuve à une température relativement stable, typiquement à une température d'environ 45°C en vue d'une utilisation de la préparation de cire à épiler préalablement préparée.

Par exemple, l'appareil 1 comprend un contrôleur configuré pour contrôler le système de chauffage 7, le système de refroidissement 8 et/ou le bloc moteur 4 en fonction de la température à l'intérieur de la cuve 2.

Typiquement, le contrôleur pilote en intensité ou en tension le système de chauffage 7, de sorte à augmenter (respectivement diminuer) la température du système de chauffage 7, par exemple d'une résistance chauffante, et par conséquent d'augmenter (respectivement de diminuer) la température de la cuve 2 donc de la préparation pour cire à épiler à l'intérieur de la cuve 2.

De plus, afin d'accélérer un refroidissement de la cuve 2, le contrôleur peut piloter le système de refroidissement 8, afin d'entrainer une diminution de la température à l'intérieur de la cuve, par exemple via un échange thermique par convention forcée au niveau des parois de la cuve 2. Typiquement, il peut s'agir d'augmenter une vitesse de rotation du ventilateur éventuel.

Afin d'accélérer le chauffage ou le refroidissement du contenu de la cuve 2, le contrôleur peut en outre piloter en tension ou en intensité le bloc moteur 4 pour augmenter la vitesse de rotation de la pâle (61,62), ce qui permet d'augmenter les échanges thermiques.

Le contrôleur peut en outre comprendre un minuteur électronique, permettant de mesurer une durée préprogrammée de phases de fonctionnement de l'appareil 1.

Le contrôleur peut être en communication avec un moyen d'alarme, par exemple un voyant lumineux, indiquant un fonctionnement ou une mise sous tension de l'appareil 1, ou un haut-parleur pouvant émettre un signal sonore, typiquement lorsque la préparation pour cire à épiler est prête à être utilisée.

L'appareil 1 peut comprendre en outre un capteur de température externe, typiquement disposé à l'extérieur de la cuve 2, par exemple sur la portion inférieure 12 de la base 10. Le capteur de température externe est configuré pour déterminer la température à l'extérieur de l'appareil 1. Dans un exemple de réalisation, un système d'avertissement peut être prévu pour donner des informations à un utilisateur de l'appareil 1 en fonction de la température à l'extérieur de l'appareil 1. Typiquement, il peut s'agir d'un écran d'affichage indiquant une masse de différents composants à introduire dans la cuve 2 pour réaliser la préparation pour cire à épiler. En effet, une masse relative des différents composants peut varier selon la température ambiante, comme cela sera décrit par la suite.

L'appareil 1 peut comprendre un système de pesée, configuré pour peser un contenu de la cuve 2. Le système de pesée peut être typiquement une balance électronique comprenant une jauge de déformation disposée sous la cuve 2 et un écran d'affichage, de sorte que l'introduction des différents composants dans la cuve 2 entraine une déformation de la jauge et un affichage d'une masse introduite sur l'écran d'affichage. Dans un autre exemple de réalisation, l'appareil 1 peut comprendre une dosette calibrée de sorte à pouvoir introduire dans la cuve une masse correcte des différents composants.

Il est également proposé ici un procédé de fabrication de cire à épiler. Le procédé de fabrication de cire à épiler peut typiquement être mis en œuvre de manière automatisée par l'appareil 1 tel que décrit précédemment, ou de manière générale par un appareil comprenant un moyen de chauffage, un système de malaxage et de mélange, un système de refroidissement pour une cuve, destinée à contenir une préparation pour cire à épiler. Par le procédé de fabrication de cire à épiler peut être mis en œuvre de manière automatisée par l'appareil 1 tel que décrit précédemment, on comprend que l'étape a) incorporation d'une préparation pour cire à épiler dans la cuve est réalisée manuellement.

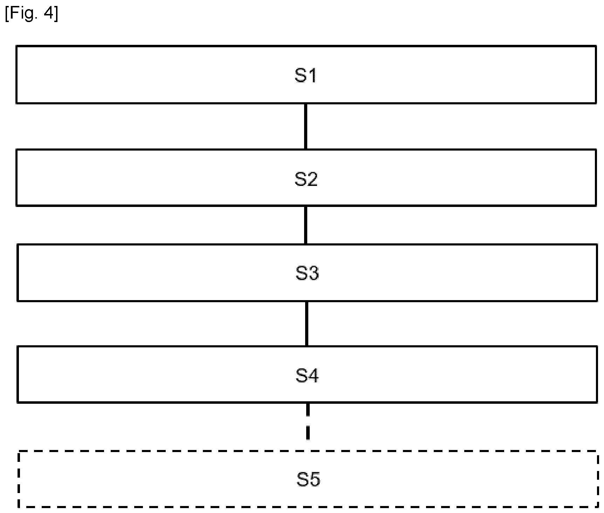

Selon un exemple de réalisation, le procédé comprend une succession d'étapes. La figure 4 illustre schématiquement la succession d'étapes du procédé. On entendra par « environ » une tolérance de plus ou moins 1%, et par « une température de l'ordre de » une tolérance de plus ou moins 1 °C. L'homme du métier comprendra que les grandeurs mesurées ne présentent pas de valeurs exactes, et que les tolérances peuvent dépendre de la précision des moyens de mesure de ces grandeurs.

Une première étape S1 du procédé de fabrication proposé comprend l'incorporation d'une préparation pour cire à épiler dans la cuve.

La préparation comprend un mélange d'une matière majoritairement composée de glucides simples (par exemple sucre ou saccharose), d'une solution acidifiante (par exemple jus de citron) et d'eau.

Selon un exemple particulier, la préparation comprend un mélange de sucre, d'eau et de jus de citron.

Typiquement, la préparation pour cire à épiler est composée d'environ 55% de sucre, 35% de jus de citron et 10% d'eau. Cependant, la composition peut varier en fonction de la température ambiante ainsi que de la température des mains de l'utilisateur par exemple. D'autres exemples de compositions sont ainsi indiqués plus loin dans la description.

Une deuxième étape S2 comprend un chauffage et un mélange simultané de la préparation pour cire à épiler jusqu'à une première température (T1). Typiquement, la première température est par exemple comprise entre 125°C et 140°C, et de préférence entre 130°C et 135°C, afin d'atteindre une dissolution totale du sucre dans l'eau et le jus de citron, et un stade de cuisson du sucre garantissant une bonne adhésion à une zone à épiler lors d'une utilisation de la préparation pour cire à épiler. La deuxième étape S2 peut typiquement être effectuée pendant une durée d'environ 15min. La durée dépend par exemple de la quantité de préparation pour cire à épiler contenue dans la cuve et d'une puissance du moyen de chauffage.

Une troisième étape S3 comprend un refroidissement forcé, c'est-à-dire artificiel et non naturel, et un mélange simultané de la préparation jusqu'à une deuxième température (T2), la deuxième température étant par exemple de l'ordre de 70°C. Le refroidissement forcé peut être effectué par le système de refroidissement de l'appareil 1 tel que décrit précédemment, ou de manière générale par tout dispositif capable d'imposer un refroidissement suffisamment rapide à la préparation pour cire à épiler contenue dans la cuve.

En dessous de la deuxième température, le mélange de la préparation s'arrête. Cela permet notamment de ne pas endommager le système de mélange, lors d'une mise en œuvre du procédé par un appareil tel que décrit précédemment, car la viscosité de la préparation peut être importante.

Une quatrième étape S4 comprend donc un refroidissement, également forcé, sans mélange de la préparation jusqu'à une troisième température (T3), la troisième température étant par exemple de l'ordre de 45°C. Lorsque la préparation atteint la troisième température, la préparation est prête à être utilisée par un utilisateur comme cire à épiler. Ainsi, la troisième température ne doit pas être trop élevée, typiquement doit être inférieure à 52°C, pour ne pas être trop liquide ni risquer d'entrainer une brûlure de l'utilisateur lors d'une application sur une zone à épiler. La troisième température ne doit pas être trop faible, afin de permettre une application correcte sur une zone à épiler, par exemple un étalement fin de la préparation de cire à épiler sur une zone à épiler avec une spatule, ou une utilisation manuelle de l'utilisateur. En effet, un refroidissement trop important de la préparation entrainerait une augmentation de la viscosité de cette préparation pouvant aller jusqu'à un durcissement empêchant son étalement.

La troisième étape S3 et la quatrième étape S4 de refroidissement forcé peuvent typiquement avoir une durée totale inférieure à 20min. La durée de refroidissement peut dépendre par exemple de la quantité de préparation pour cire à épiler contenue dans la cuve, d'une puissance du moyen de refroidissement, ou de la température ambiante.

De préférence, le procédé comprend également une cinquième étape S5 de maintien de la préparation à la troisième température, lorsque la troisième température est atteinte suite au refroidissement forcé. Cette étape peut être facilitée par la présence d'un thermostat. Un maintien de la préparation à la troisième température permet de conserver une température adéquate de la cire à épiler pendant toute une durée d'utilisation, typiquement supérieure à 5 min, de préférence inférieure à 60 min, et de préférence encore de l'ordre de 30 min. Une fois prête, la cire peut être récupérée dans la cuve, par exemple avec un ustensile de manipulation (type spatule) voire directement avec les doigts, pour être appliquée sur la peau à épiler. Le temps d'application est généralement inférieur à 5 min.

Après utilisation de la préparation pour cire à épiler, il est possible de nettoyer la cuve.

De préférence, le procédé décrit précédemment peut comprendre un contrôle continu de la température à l'intérieur de la cuve. Dans ce mode de réalisation, la succession des étapes de chauffage de la préparation S2 et de refroidissement forcé de la préparation (S3, S4) peuvent être effectuées de manière automatique en fonction de ladite température à l'intérieur de la cuve.

Typiquement, un contrôle continu de la température à l'intérieur de la cuve peut avoir lieu si le procédé de fabrication est mis en œuvre de manière avantageuse par un appareil de fabrication de cire à épiler 1 tel que décrit précédemment. Un contrôleur relié à un capteur de température interne peut piloter les moyens de chauffage, de mélange et de refroidissement pour mettre en œuvre successivement les étapes du procédés lorsqu'une température cible de transition entre chaque étape est atteinte.

Comme indiqué en préambule, la composition de la cire à épiler peut varier selon la température ambiante, typiquement une température régnant dans une salle où a lieu une épilation avec la cire à épilée fabriquée lors d'une mise en œuvre du procédé. La composition de la préparation incorporée dans la cuve peut être contrôlée par une pesée préalable des composants, ou par l'utilisation de dosettes calibrées, comme suggéré précédemment.

Typiquement, pour une température ambiante comprise environ entre 20°C et 23°C, la préparation utilisée pour la première étape peut être un mélange comprenant 55% de sucre, 10% d'eau et 35% de jus de citron. Par exemple, pour obtenir 120g de cire à épiler, on peut incorporer dans la cuve 110g de sucre, 70g de jus de citron et 20g d'eau.

De même, pour une température ambiante comprise environ entre 24°C et 27°C, la préparation utilisée pour la première étape peut être un mélange comprenant 55% de sucre, 13% d'eau et 32% de jus de citron. Par exemple, pour obtenir 120g de cire à épiler, on peut incorporer dans la cuve 110g de sucre, 64g de jus de citron et 26g d'eau.

Enfin, pour une température ambiante comprise environ entre 28°C et 30°C, la préparation utilisée pour la première étape peut être un mélange comprenant 55% de sucre, 17% d'eau et 28% de jus de citron. Par exemple, pour obtenir 120g de cire à épiler, on peut incorporer dans la cuve 110g de sucre, 56g de jus de citron et 34g d'eau.

Par ailleurs, pour une température ambiante comprise environ entre 23°C et 24°C, on peut utiliser un mélange comprenant 55% de sucre, une proportion de jus de citron choisie entre 32% et 35%, et le reste d'eau.

Pour une température ambiante comprise environ entre 27°C et 28°C, on peut utiliser un mélange comprenant 55% de sucre, une proportion de jus de citron choisie entre 28% et 32%, et le reste d'eau.

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée de l'appareil et du procédé présentés.

### REFERENCES BIBLIOGRAPHIQUES

FR2777458, FR2541894, EP1568294

## Revendications

1. Appareil pour fabrication de cire à épiler (1), comprenant :
- une cuve (2) destinée à recevoir une préparation pour cire à épiler ;
- un couvercle (3) prévu pour fermer la cuve (2) ;
- un bloc moteur (4) et un arbre d'entrainement (5) configuré pour être entrainé en rotation par ledit bloc moteur (4) ;
- au moins une pâle (61,62) prévue pour être mise en rotation par l'arbre d'entrainement (5), l'au moins une pâle (61,62) étant agencée dans la cuve (2) pour permettre un mélange de la préparation pour cire à épiler ;
- un système de chauffage (7) configuré pour un chauffage de la cuve (2) ; et
- un système de refroidissement (8) configuré pour un refroidissement de la cuve (2) **caractérisé en ce que** l'appareil comprend en outre un capteur de température interne permettant de déterminer la température à l'intérieur de la cuve (2), et un contrôleur configuré pour contrôler le système de chauffage (7), le système de refroidissement (8) et/ou le bloc moteur (4) en fonction de la température à l'intérieur de la cuve (2), l'appareil mettant en œuvre les étapes successives suivantes d'un procédé de fabrication de cire à épiler:
a) incorporation d'une préparation pour cire à épiler dans la cuve, la préparation comprenant un mélange d'une matière majoritairement composée de glucides simples, d'une solution acidifiante et d'eau ;
b) chauffage et mélange de ladite préparation jusqu'à une première température, la première température étant comprise entre 125°C et 140°C, et de préférence entre 130°C et 135°C,
c) refroidissement forcé et mélange de la préparation jusqu'à une deuxième température, la deuxième température étant de l'ordre de 70°C,
d) refroidissement forcé sans mélange de la préparation jusqu'à une troisième température, la troisième température étant de l'ordre de 45°C.

2. Appareil selon la revendication 1, dans lequel la cuve (2) comprend un fond et des parois latérales, le système de chauffage (7) étant agencé pour un chauffage du fond de la cuve (2) et le système de refroidissement (8) étant agencé pour un refroidissement des parois latérales de la cuve (2).

3. Appareil selon l'une quelconque des revendications 1 et 2, dans lequel la cuve (2) et/ou l'au moins une pâle (61,62) sont amovibles.

4. Appareil selon l'une quelconque des revendications 1 à 3, comprenant en outre une base (10) ayant une portion inférieure (12) et une portion supérieure (11) couplée à la portion inférieure (12), la portion supérieure (11) étant destinée à recevoir la cuve (2) de sorte qu'un espace de refroidissement est défini entre une surface latérale extérieure de la cuve (2) et une surface intérieure de la portion supérieure (11).

5. Appareil selon la revendication 4, dans lequel la portion inférieure (12) est formée par une première coque et la portion supérieure (11) est formée par une deuxième coque venant prolonger la première coque.

6. Appareil selon quelconque des revendications 4 et 5, dans lequel la portion inférieure (12) et/ou la portion supérieure (11) comprend des ouvertures (13,14) configurées pour permettre une circulation d'air dans l'espace de refroidissement.

7. Appareil selon l'une quelconque des revendications 1 à 6, dans lequel le couvercle (3) comprend une pluralité d'ouvertures (31) pour une circulation d'air entre l'intérieur de la cuve (2) et l'extérieur, et un système de blocage (32) configuré pour verrouiller une fermeture de la cuve (2).

8. Appareil selon l'une quelconque des revendications précédentes, comprenant en outre un élément de protection thermique (9) agencé pour isoler thermiquement le système de chauffage (7) du bloc moteur (4) et/ou du système de refroidissement (8).

9. Appareil selon l'une quelconque des revendications 1 à 8, dans lequel le système de refroidissement (8) comprend un ventilateur, ledit ventilateur étant agencé pour diriger de l'air en direction de la cuve (2).

10. Appareil selon l'une quelconque des revendications 1 à 9, comprenant en outre un capteur de température externe permettant de déterminer la température à l'extérieur de l'appareil (1), et un système d'avertissement prévu pour donner des informations à un utilisateur de l'appareil (1) en fonction de la température à l'extérieur de l'appareil.

11. Appareil selon l'une quelconque des revendications 1 à 10, dans lequel l'étape d) est suivie d'une étape e) de maintien de la préparation à la troisième température.

12. Appareil selon l'une quelconque des revendications 1 à 11, dans lequel l'étape b) est effectuée pendant une durée d'environ 15min.

13. Appareil selon l'une quelconque des revendications 1 à 12, dans lequel les étapes c) et d) ont une durée totale inférieure à 20min.

14. Appareil selon l'une quelconque des revendications 1 à 13, qui comprend un contrôle continu de la température à l'intérieur de la cuve (2), la succession des étapes b) à d) étant automatiquement effectuée en fonction de ladite température à l'intérieur de la cuve (2).

15. Appareil selon l'une quelconque des revendications 1 à 14, dans lequel la préparation utilisée pour l'étape a) est un mélange comprenant :
- 55% de sucre, 10% d'eau et 35% de jus de citron pour une température ambiante comprise entre 20°C et 23°C ; ou
- 55% de sucre, 13% d'eau et 32% de jus de citron pour une température ambiante comprise entre 24°C et 27°C ; ou
- 55% de sucre, 17% d'eau et 28% de jus de citron pour une température ambiante comprise entre 28°C et 30°C.
